# EUROPEAN PATENT APPLICATION

(11) **EP 4 434 577 A1**
(43) Date of publication of application: **25.09.2024**
(21) Application number: 23201109.8
(22) Date of filing: 02.10.2023
(51) Int. Cl.: A61N 5/10

(54) **PARTICLE BEAM IRRADIATION SYSTEM AND PARTICLE BEAM IRRADIATION METHOD**

(30) Priority: 22.03.2023 JP 2023045073
(71) Applicant: Hitachi, Ltd., Tokyo 100-8280 (JP)
(72) Inventor: EGUCHI, Mina, Tokyo, 100-8280 (JP); NOMURA, Takuya, Tokyo, 100-8280 (JP)
(74) Representative: MERH-IP Matias Erny Reichl Hoffmann Patentanwälte PartG mbB

(57) **Abstract**

A particle beam irradiation system (100) according to an aspect of the present invention includes two or more charged particle beam generation apparatuses (1) capable of operating independently of each other, a beam transport line (2) that transports charged particle beams generated by the charged particle beam generation apparatuses (1), and two or more beam irradiation apparatuses (3) to which the charged particle beams are transported through the beam transport line (2). Any one of the beam irradiation apparatuses (3) is configured such that the charged particle beams from a plurality of the charged particle beam generation apparatuses (1) can be transported thereto, and the charged particle beams are simultaneously transported from the plurality of charged particle beam generation apparatuses (1) to corresponding ones of the different beam irradiation apparatuses (3).

## Description

### BACKGROUND OF THE INVENTION

### 1. Field of the Invention

The present invention relates to a particle beam irradiation system and a particle beam irradiation method.

### 2. Description of the Related Art

In particle beam therapy, in order to irradiate a predetermined position with a particle beam as planned, the position of a patient is set before the irradiation. After the position has been set, the patient is not allowed to move and needs to keep the same posture until the beam irradiation is completed. Further, a particle beam cannot be transported from one accelerator to a plurality of irradiation rooms (beam irradiation apparatuses) simultaneously. Therefore, in a case where an accelerator to be used is transporting a particle beam to another irradiation room at the time when the position of the patient has been set, the patient needs to wait until the beam irradiation is completed in the other irradiation room. This decreases the efficiency and also places a burden on the patient. Therefore, there has been invented a method of performing beam irradiation in a plurality of irradiation rooms at the same time.

For example, JP-2005-302734-A discloses an accelerator system for accelerating an ion beam by a post-accelerator including a synchrotron, and supplying a high-energy ion beam to a plurality of irradiation rooms to perform therapy. In the accelerator system, one post-accelerator and a plurality of treatment rooms (irradiation rooms) are connected to each other through a transport line.

### SUMMARY OF THE INVENTION

As disclosed in JP-2005-302734-A, the method capable of performing the beam irradiation in a plurality of irradiation rooms at the same time has already been invented. However, a plurality of accelerators cannot be driven independently of each other, and an accelerator from which a particle beam is to be transported is allocated to each irradiation room. Therefore, if one of the accelerators fails, one or more corresponding irradiation rooms are also disabled. That is, if an accelerator fails, there is always an irradiation room that cannot be used, and thus, the throughput upon failure of the accelerator drops significantly in the above-mentioned method.

In view of the situation described above, there has been demanded a method capable of performing simultaneous irradiation in a plurality of irradiation rooms and also minimizing degradation of the throughput when an accelerator fails.

In order to solve the problem described above, according to an aspect of the present invention, there is provided a particle beam irradiation system including two or more charged particle beam generation apparatuses capable of operating independently of each other, a beam transport line that transports charged particle beams generated by the charged particle beam generation apparatuses, and/or two or more beam irradiation apparatuses to which the charged particle beams are transported through the beam transport line. In the particle beam irradiation system, any one of the beam irradiation apparatuses is configured such that the charged particle beams from a plurality of the charged particle beam generation apparatuses can be transported thereto, and the charged particle beams are simultaneously transported from the plurality of charged particle beam generation apparatuses to corresponding ones of the different beam irradiation apparatuses.

According to at least the aspect of the present invention, simultaneous irradiation can be performed by a plurality of beam irradiation apparatuses, and throughput reduction upon failure of a charged particle beam generation apparatus can be minimized.

Problems, configurations, and advantageous effects other than those described above will be apparent from the following description of an embodiment.

### BRIEF DESCRIPTION OF THE DRAWINGS

FIG. 1 is a schematic view depicting a particle beam irradiation system according to an embodiment of the present invention;
FIG. 2 is a block diagram depicting an example of a minimum configuration of the particle beam irradiation system according to the embodiment of the present invention;
FIG. 3 is a block diagram depicting another example of the configuration of the particle beam irradiation system according to the embodiment of the present invention; and
FIG. 4 is a block diagram depicting an example of a hardware configuration of an overall controller according to the embodiment of the present invention.

### DESCRIPTION OF THE PREFERRED EMBODIMENT

In the following, an example of a mode for carrying out the present invention (hereinafter referred to as an "embodiment") is described with reference to the accompanying drawings. In the present specification and the accompanying drawings, identical components or components having substantially identical functions are denoted by identical reference characters, and overlapping description thereof is omitted suitably. Further, in a case where a plurality of components have identical or similar functions, different suffixes are sometimes added to the same reference character in the description. In addition, in a case where there is no necessity to distinguish these components from each other, their suffixes are sometimes omitted in the description.

### <First Embodiments

### [Overview of particle beam irradiation system]

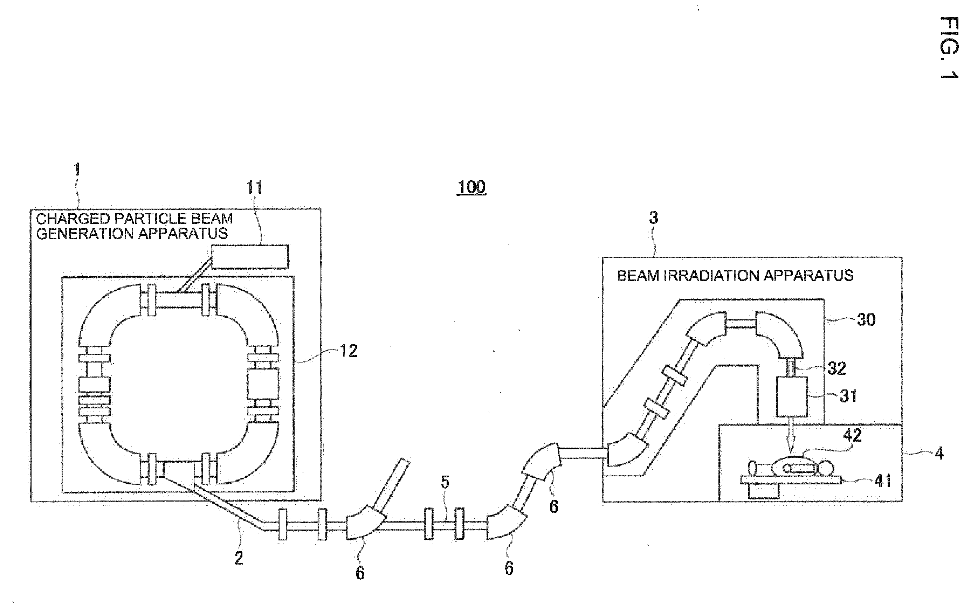

First, a rough configuration of a particle beam irradiation system according to an embodiment of the present invention is described with reference to FIG. 1.

FIG. 1 is a schematic view depicting the particle beam irradiation system according to the present embodiment. As depicted in FIG. 1, a particle beam irradiation system 100 according to the present embodiment includes a charged particle beam generation apparatus 1, a beam transport line 2, and a beam irradiation apparatus 3. Generally, a particle beam irradiation system includes a charged particle beam generation apparatus, a beam transport line, and a beam irradiation apparatus.

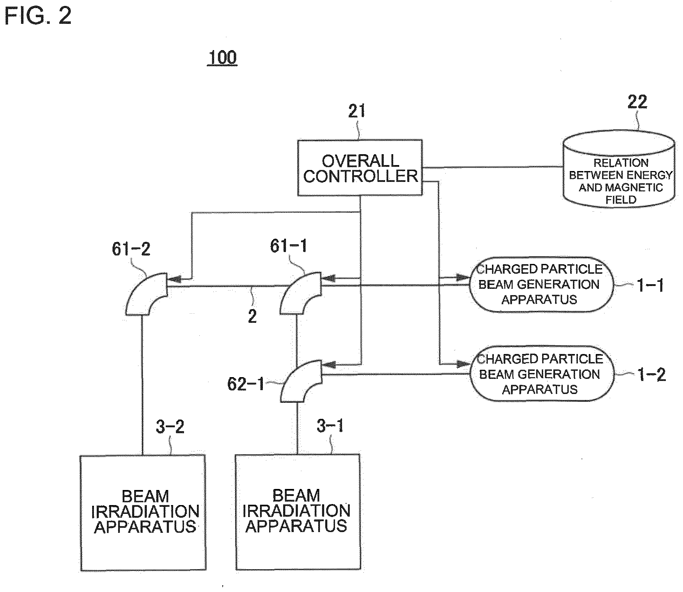

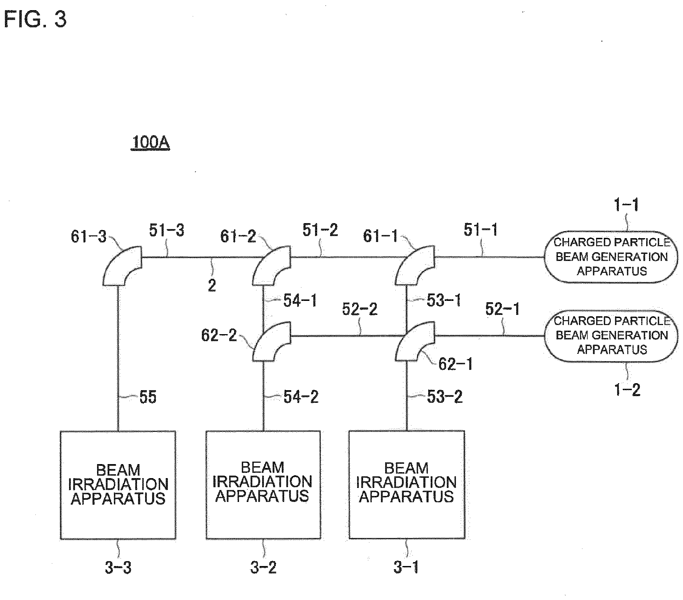

The charged particle beam generation apparatus 1 includes an ion source (not depicted), a pre-accelerator 11, and a circular accelerator (synchrotron) 12, and generates a charged particle beam 32. Although, in the present embodiment, a synchrotron is described as an example of the circular accelerator 12, another accelerator such as a cyclotron may be used. The ion source is connected to the upstream side of the pre-accelerator 11, and the circular accelerator 12 is connected to the downstream side of the pre-accelerator 11. As depicted in FIGS. 2 and 3 described later, the particle beam irradiation system 100 according to the present embodiment includes a plurality of charged particle beam generation apparatuses 1.

The beam transport line 2 is connected to the downstream side of the charged particle beam generation apparatus 1, and connects the charged particle beam generation apparatus 1 and the beam irradiation apparatus 3 to each other through a vacuum pipe 5. A plurality of bending magnets 6 are provided on the vacuum pipe 5. The charged particle beam 32 generated by the charged particle beam generation apparatus 1 is introduced to the respective beam irradiation apparatuses 3 in irradiation rooms by the bending magnets 6 through the beam transport line 2.

Each of the bending magnets 6 is arranged such that a pair of electromagnets face each other across the vacuum pipe 5. A magnetic field is generated between the pair of electromagnets according to electric energy applied to the bending magnet 6 and changes the advancing direction of the charged particle beam passing between the electromagnets. The vacuum pipe 5 at positions where the bending magnets 6 are arranged has such a structure as to enable the charged particle beam to either advance straightforwardly or bend in a perpendicular direction. Thus, when the intensity of the magnetic field to be applied to the bending magnet 6 is controlled, the charged particle beam can be made to advance straightforwardly or bend in the perpendicular direction.

As depicted in FIGS. 2 and 3 described later, a plurality of beam irradiation apparatuses 3 can be provided in the particle beam irradiation system 100 according to the present embodiment. In a case where a plurality of beam irradiation apparatuses 3 are provided, the charged particle beam 32 is introduced to corresponding one of the beam irradiation apparatuses 3 when the bending magnet 6 which is disposed at a suitable position from among the plurality of bending magnets 6 on the beam transport line 2 is controlled.

The beam irradiation apparatus 3 is an apparatus for irradiating an affected part of a patient 42 who is standing by in a treatment room 4, with the charged particle beam 32 transported through the beam transport line 2. As depicted in FIG. 1, the beam irradiation apparatus 3 according to the present embodiment includes a treatment table 41 on which the patient 42 is to be placed thereon, an irradiation nozzle (nozzle device) 31, and a rotation gantry 30. It is to be noted that a fixed irradiation type (two-dimensional plane type) beam irradiation apparatus that does not include the rotation gantry 30 may be used.

### [Minimum configuration of particle beam irradiation system]

Next, a minimum configuration of the particle beam irradiation system according to the present embodiment is described with reference to FIG. 2.

FIG. 2 is a block diagram depicting an example of a minimum configuration of the particle beam irradiation system 100. The minimum configuration of the particle beam irradiation system includes two charged particle beam generation apparatuses 1-1 and 1-2 and two beam irradiation apparatuses 3-1 and 3-2 as depicted in FIG. 2. The beam irradiation apparatuses 3-1 and 3-2 are installed in the separate treatment rooms 4 (FIG. 1).

The charged particle beam generation apparatuses 1-1 and 1-2 have the same structure as that of the charged particle beam generation apparatus 1 depicted in FIG. 1, and the beam irradiation apparatuses 3-1 and 3-2 have the same structure as that of the beam irradiation apparatus 3 depicted in FIG. 1. The charged particle beam generation apparatuses 1-1 and 1-2 can be driven independently of each other, and even if one of the charged particle beam generation apparatuses 1-1 and 1-2 fails, the other one of the charged particle beam generation apparatuses 1-1 and 1-2 can operate. That is, the two charged particle beam generation apparatuses are physically separated apparatuses, and they are controlled so as not to rely on each other. Further, the types of ion sources handled by the two charged particle beam generation apparatuses 1-1 and 1-2 are not limited to particular ones, and may be the same nuclear species or different nuclear species.

In the present embodiment, charged particle beams from the charged particle beam generation apparatuses 1-1 and 1-2 can be transported to either the beam irradiation apparatus 3-1 or 3-2. However, charged particle beams from a plurality of charged particle beam generation apparatuses are not simultaneously transported to one beam irradiation apparatus.

Further, in the particle beam irradiation system 100 depicted in FIG. 2, bending magnets 61-1 and 61-2 and a bending magnet 62-1 are provided on the beam transport line 2. Each of the bending magnets 61-1 and 61-2 and the bending magnet 62-1 has the same structure as that of the bending magnets 6 of FIG. 1.

In the particle beam irradiation system 100, a charged particle beam generated by the charged particle beam generation apparatus 1-1 can be transported to the beam irradiation apparatus 3-1 through the bending magnets 61-1 and 62-1 on the beam transport line 2. Further, the charged particle beam generated by the charged particle beam generation apparatus 1-1 can be transported to the beam irradiation apparatus 3-2 through the bending magnets 61-1 and 61-2 on the beam transport line 2. Meanwhile, a charged particle beam generated by the charged particle beam generation apparatus 1-2 can be transported to the beam irradiation apparatus 3-1 through the bending magnet 62-1 on the beam transport line 2.

The particle beam irradiation system 100 includes an overall controller 21 that outputs a control signal to the charged particle beam generation apparatuses 1-1 and 1-2 and the bending magnets 61-1, 61-2, and 62-1 to control operations of the respective charged particle beam generation apparatuses and the respective bending magnets.

A storage unit 22 in which a relation between energy and a magnetic field is stored is connected to the overall controller 21. The acceleration of a charged particle depends upon the intensity of the magnetic field. Generally, in a case where a charged particle has an equal charged amount, as the magnetic field intensity increases, the energy that the charged particle receives from the magnetic field increases, and the kinetic energy of the charged particle (acceleration) increases.

Further, the overall controller 21 outputs a command to an undepicted controller provided for the bending magnet. The controller for the bending magnet may be provided for each bending magnet or provided for each group (for example, a group determined taking a transport path of a charged particle beam into consideration) of a plurality of bending magnets. According to the command from the overall controller 21, the controller for the bending magnet supplies electric energy to the corresponding bending magnet to generate a magnetic field and perform switching of the beam transport.

In the example depicted in FIG. 2, the charged particle beam generated by the charged particle beam generation apparatus 1-1 changes its advancing direction to a perpendicular direction at the bending magnet 61-1 and is transported to the beam irradiation apparatus 3-1 through the bending magnet 62-1. Further, the charged particle beam generated by the charged particle beam generation apparatus 1-1 is transported to the beam irradiation apparatus 3-2 through the bending magnet 61-1 and the bending magnet 61-2. Meanwhile, the charged particle beam generated by the charged particle beam generation apparatus 1-2 changes its advancing direction to a perpendicular direction at the bending magnet 62-1 and is transported to the beam irradiation apparatus 3-1.

Further, the particle beam irradiation system 100 includes an undepicted controller for controlling operations of the beam irradiation apparatuses 3-1 and 3-2. This controller controls the operations of the beam irradiation apparatuses 3-1 and 3-2 according to an engineer's command or on the basis of a condition set in advance, to irradiate a target position of a patient 42 on the treatment table 41 in each treatment room 4 with a charged particle beam.

In this manner, in the present embodiment, a first charged particle beam can be transported from a first charged particle beam generation apparatus to a first beam irradiation apparatus, and concurrently with this, a second charged particle beam can be transported from a second charged particle beam generation apparatus to a second beam irradiation apparatus through a shared beam transport line.

In the present embodiment configured as described above, since the respective charged particle beam generation apparatuses have separate transport paths of charged particle beams, charged particle beams can be applied concurrently by a plurality of beam irradiation apparatuses (treatment rooms).

### [Another configuration of particle beam irradiation system]

The number of charged particle beam generation apparatuses, the number of beam irradiation apparatuses, the arrangement of them, and so forth differ among different facilities. Here, as a typical example, a particle beam irradiation system that includes two charged particle beam generation apparatuses and three beam irradiation apparatuses is described with reference to FIG. 3.

FIG. 3 is a block diagram depicting another example of the configuration of the particle beam irradiation system according to the present embodiment. A particle beam irradiation system 100A depicted in FIG. 3 typically includes two charged particle beam generation apparatuses 1-1 and 1-2 and three beam irradiation apparatuses 3-1, 3-2, and 3-3. The beam irradiation apparatuses 3-1 to 3-3 are installed in the separate treatment rooms 4 (FIG. 1).

The charged particle beam generation apparatuses 1-1 and 1-2 have the same structure as that of the charged particle beam generation apparatus 1 depicted in FIG. 1, and the beam irradiation apparatuses 3-1, 3-2, and 3-3 have the same structure as that of the beam irradiation apparatus 3 depicted in FIG. 1. As in the case depicted in FIG. 2, the charged particle beam generation apparatuses 1-1 and 1-2 can be driven independently of each other, and even if one of the charged particle beam generation apparatuses 1-1 and 1-2 fails, the other charged particle beam generation apparatus can operate. Further, the types of ion sources handled by the two charged particle beam generation apparatuses 1-1 and 1-2 are not limited to particular ones, and may be the same nuclear species or different nuclear species.

The particle beam irradiation system 100A depicted in FIG. 3 further includes, on the beam transport line 2, bending magnets 61-1, 61-2, and 61-3 and bending magnets 62-1 and 62-2. Each of the bending magnets 61-1 to 61-3 and 62-1 to 62-2 has the same structure as that of the bending magnet 6 of FIG. 1. In the following description, in a case where there is no necessity to distinguish the bending magnets from one another, they are simply referred to as bending magnets 61.

The particle beam irradiation system 100A is configured such that charged particle beams from the plurality of charged particle beam generation apparatuses 1-1 and 1-2 can be transported to any one of the beam irradiation apparatuses 3-1, 3-2, and 3-3. In the example of FIG. 3, it is possible to transport a charged particle beam from the charged particle beam generation apparatus 1-1 to the beam irradiation apparatuses 3-1, 3-2, and 3-3, and transport a charged particle beam from the charged particle beam generation apparatus 1-2 to the beam irradiation apparatuses 3-1 and 3-2. However, charged particle beams from a plurality of charged particle beam generation apparatuses are not simultaneously transported to one beam irradiation apparatus.

For example, a charged particle beam generated by the charged particle beam generation apparatus 1-1 can be transported to the beam irradiation apparatus 3-1 through a vacuum pipe 51-1, the bending magnet 61-1, a vacuum pipe 53-1, the bending magnet 62-1, and a vacuum pipe 53-2 on the beam transport line 2. Further, the charged particle beam generated by the charged particle beam generation apparatus 1-1 can be transported to the beam irradiation apparatus 3-2 through the vacuum pipe 51-1, the bending magnet 61-1, a vacuum pipe 51-2, the bending magnet 61-2, a vacuum pipe 54-1, the bending magnet 62-2, and a vacuum pipe 54-2 on the beam transport line 2. Further, the charged particle beam generated by the charged particle beam generation apparatus 1-1 can be transported to the beam irradiation apparatus 3-3 through the vacuum pipe 51-1, the bending magnet 61-1, the vacuum pipe 51-2, the bending magnet 61-2, a vacuum pipe 51-3, the bending magnet 61-3, and a vacuum pipe 55 on the beam transport line 2.

A charged particle beam generated by the charged particle beam generation apparatus 1-2 can be transmitted to the beam irradiation apparatus 3-1 through a vacuum pipe 52-1, the bending magnet 62-1, and the vacuum pipe 53-2 on the beam transport line 2. Further, the charged particle beam generated by the charged particle beam generation apparatus 1-2 can be transported to the beam irradiation apparatus 3-2 through the vacuum pipe 52-1, the bending magnet 62-1, a vacuum pipe 52-2, the bending magnet 62-2, and the vacuum pipe 54-2 on the beam transport line 2. It is to be noted that the vacuum pipe 52-2 and the vacuum pipe 55 may be connected to each other.

As described above, the particle beam irradiation system according to the present embodiment includes two or more charged particle beam generation apparatuses that can be driven independently of each other, a beam transport line that transports charged particle beams generated by the charged particle beam generation apparatuses, and two or more beam irradiation apparatuses to which the charged particle beams are transported through the beam transport line. Further, the particle beam irradiation system is configured such that charged particle beams from a plurality of charged particle beam generation apparatuses can be transported to any one of the beam irradiation apparatuses, and the charged particle beams from the plurality of charged particle beam generation apparatuses are simultaneously transported to corresponding ones of the different beam irradiation apparatuses.

According to the present embodiment configured as described above, the charged particle beam generation apparatus from which a charged particle beam can be transmitted to the beam irradiation apparatus (treatment room) is not limited to a particular one, and a plurality of charged particle beam generation apparatuses are configured to be able to operate independently of each other. Hence, a plurality of beam irradiation apparatuses can perform simultaneous irradiation, and the number of patients who can receive treatment per hour can be increased. Further, according to the present embodiment, reduction of the throughput upon failure of a charged particle beam generation apparatus can be suppressed to the minimum.

Further, in the particle beam irradiation system 100A described above, the advancing direction of the first charged particle beam and the advancing direction of the second charged particle beam sometimes intersect in a vacuum pipe at a position (for example, at the bending magnet 62-1) where a bending magnet is arranged on the shared beam transport line.

Here, it does not matter whether, at a portion where charged particle beams transported from the two charged particle beam generation apparatuses 1-1 and 1-2 may possibly intersect, beam transport lines through which the intersecting charged particle beams pass are independent of each other. For example, in a case where a charged particle beam is transported from the charged particle beam generation apparatus 1-1 to the beam irradiation apparatus 3-1 and where another charged particle beam is transported from the charged particle beam generation apparatus 1-2 to the beam irradiation apparatus 3-2, the charged particle beams intersect at the position of the bending magnet 62-1. In such a case, the vacuum pipe 53-1 and the vacuum pipe 52-1 that are to be used may be independent of each other or may be common to each other. In other words, the vacuum pipe 53-1 and the vacuum pipe 52-1 may be independent of each other or may be connected to each other.

Also, the vacuum pipe 52-1 and the vacuum pipe 53-2 that are used when a charged particle beam is to be transported from the charged particle beam generation apparatus 1-2 to the beam irradiation apparatus 3-1 may be independent of each other or may be common to each other.

Also in a case where a common beam transport line is used, it is possible to transport a charged particle beam from the charged particle beam generation apparatus 1-2 to the beam irradiation apparatus 3-2 while transporting a charged particle beam from the charged particle beam generation apparatus 1-1 to the beam irradiation apparatus 3-1. In a situation in which charged particle beams transported from the charged particle beam generation apparatuses 1-1 and 1-2 pass a bending magnet 61 (for example, the bending magnet 62-1) at the same time, it is not necessary to apply a magnetic field at the bending magnet 61 to bend the charged particle beams in a perpendicular direction. Therefore, at the bending magnet 61 where the two charged particle beams intersect, each of the two charged particle beams advances straightforwardly. Also, the probability that charged particle beams that intersect may collide with each other (probability that a particle exists at a certain place) is negligible. It is most preferable that two charged particle beams intersect orthogonally (perpendicularly).

In this manner, it is possible to simultaneously transport charged particle beams from two charged particle beam generation apparatuses to a plurality of beam irradiation apparatuses.

Further, in a case where the charged particle beam generation apparatus 1-2 fails, in the configuration of FIG. 3, it is possible to continuously use the beam irradiation apparatuses 3-1 and 3-2 when the charged particle beam generation apparatus 1-1 is used. Therefore, reduction of the throughput when the charged particle beam generation apparatus 1-2 fails can be minimized.

### [Hardware configuration of overall controller]

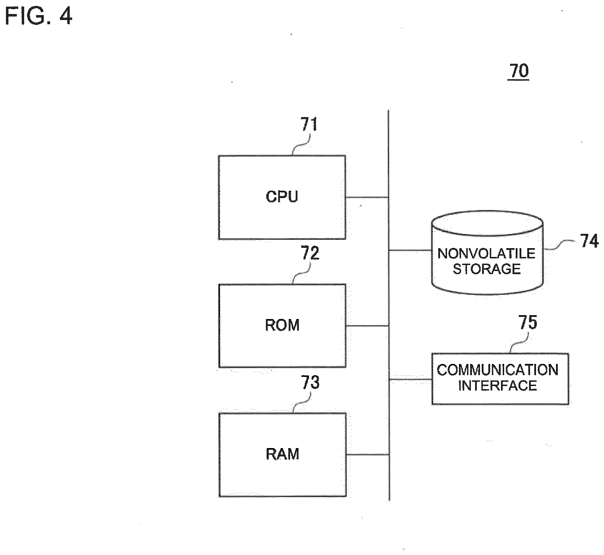

Next, a configuration (hardware configuration) of a control system of the overall controller 21 included in the particle beam irradiation system 100 depicted in FIGS. 1 and 2 is described with reference to FIG. 4.

FIG. 4 is a block diagram depicting an example of a hardware configuration of the overall controller 21. A computing machine 70 depicted in FIG. 4 is hardware used as what is generally called a computer.

The computing machine 70 includes a central processing unit (CPU) 71, a read only memory (ROM)72, a random access memory (RAM) 73, a nonvolatile storage 74, and a communication interface 75 that are individually connected to a bus.

The CPU 71 reads out, from the ROM 72, program codes of software for implementing various functions relating to the present embodiment, loads the program codes into the RAM 73, and executes the program codes. Otherwise, the CPU 71 may read out the program codes directly from the ROM 72 and execute the program codes as they are. It is to be noted that the computing machine 70 may include a processing device such as a micro-processing unit (MPU) in place of the CPU 71.

Variables, parameters, and so forth generated during the execution of computation processing by the CPU 71 are temporarily written into the RAM 73. It is to be noted that, in a case where the RAM 73 includes a nonvolatile medium, various kinds of information such as temporary communication path information may be stored in the RAM 73.

The beam transport switching function of the overall controller 21 is implemented by the CPU 71 reading out a program for implementing the function, from the ROM 72, and executing the program. For example, when the overall controller 21 receives a switching command for a charged particle beam generation apparatus and a beam irradiation apparatus from an engineer, the overall controller 21 executes the beam transport switching on the basis of the details of the command.

For example, a hard disk drive (HDD), a solid state drive (SSD), a flexible disk, an optical disk, a magnetooptical disk, a compact disk (CD)-ROM, a compact disk readable (CD-R), a nonvolatile memory card, and so forth can be used as the nonvolatile storage 74. In the nonvolatile storage 74, not only an operating system (OS) and various parameters but also a program and so forth for operating the computing machine 70 are recorded.

The program is stored in the form of computer-readable program codes, and the CPU 71 sequentially executes operations according to the program codes. That is, the ROM 72 or the nonvolatile storage 74 is used as an example of a computer-readable non-transitory recording medium in which a program to be executed by a computer is stored.

Although the overall controller 21 and the storage unit 22 are depicted as separate blocks in FIG. 2, the function of the storage unit 22 may be implemented by the nonvolatile storage 74.

The communication interface 75 includes, for example, a communication device for controlling communication to be performed with another apparatus. The other apparatus may be, for example, a user interface that accepts a command from an engineer, a controller for a bending magnet, a charged particle beam generation apparatus, or the like.

As a network for which the communication interface 75 performs communication control, for example, a path for serial communication of a multi-drop form such as RS-485 and a communication path that provides a plurality of topologies such as Ethernet (registered trademark) are available. As the communication path that provides a plurality of topologies, a local area network (LAN) or a wide area network (WAN) that are wired communication paths, a radio area network (RAN) that is a wired communication path, and so forth are available. Further, as the network for which the communication interface 75 performs communication control, a wireless network such as Wi-Fi (registered trademark), a wireless network in a wireless communication infrastructure, and so forth are available, for example.

Also, the controller for the bending magnet and the controller for the beam irradiation apparatus can have a hardware configuration similar to that of the example depicted in FIG. 4. In this case, in the controller for the bending magnet, the CPU 71 executes a computer program for implementing a function of changing the advancing direction of a beam, to implement the function. Meanwhile, in the controller for beam irradiation, the CPU 71 executes a computer program for implementing a beam irradiation function (beam irradiation position control and so forth), to implement the function.

Further, the present invention is not limited to the embodiment described above. Needless to say, various other applications and modifications can be made without departing the subject matter of the present invention described in the claims. For example, while the specific configuration of the above-mentioned embodiment has been described in detail in order to facilitate the understanding of the present invention, an embodiment of the present invention is not necessarily limited to the embodiment that includes all of the components described above. Also, another component can be added to the configuration of the above-mentioned embodiment, or replacement or deletion of some components can be made thereto.

Further, control lines and information lines that are considered to be necessary for describing the above-mentioned embodiment are exemplified in the above description, and all control lines or information lines of products are not necessarily depicted. It may be considered that almost all components are connected to one another in practice.

Further, such terms as "orthogonal" and "perpendicular" used in the present specification are not limited to terms that signify only "orthogonal" and "perpendicular" in a strict sense, and include not only the terms signifying "orthogonal" and "perpendicular" in a strict sense but also terms signifying "substantially orthogonal" and "substantially perpendicular" in a range in which their functions can be demonstrated.

## Claims

1. A particle beam irradiation system comprising:
two or more charged particle beam generation apparatuses capable of operating independently of each other;
a beam transport line that transports charged particle beams generated by the charged particle beam generation apparatuses; and
two or more beam irradiation apparatuses to which the charged particle beams are transported through the beam transport line, wherein
any one of the beam irradiation apparatuses is configured such that the charged particle beams from a plurality of the charged particle beam generation apparatuses can be transported thereto, and the charged particle beams are simultaneously transported from the plurality of charged particle beam generation apparatuses to corresponding ones of the different beam irradiation apparatuses.

2. The particle beam irradiation system according to claim 1, wherein
an advancing direction of a first charged particle beam from a first charged particle beam generation apparatus to a first beam irradiation apparatus and an advancing direction of a second charged particle beam from a second charged particle beam generation apparatus to a second beam irradiation apparatus intersect in a vacuum pipe at a position where a bending magnet is arranged on the beam transport line.

3. The particle beam irradiation system according to claim 1 or 2, wherein
the charged particle beam generation apparatuses have different ion sources.

4. A particle beam irradiation method performed by a particle beam irradiation system that includes two or more charged particle beam generation apparatuses capable of operating independently of each other, a beam transport line that transports charged particle beams generated by the charged particle beam generation apparatuses, and two or more beam irradiation apparatuses to which the charged particle beams are transported through the beam transport line, the method comprising:
with any one of the beam irradiation apparatuses configured such that the charged particle beams from a plurality of the charged particle beam generation apparatuses can be transported thereto, simultaneously transporting the charged particle beams from the plurality of charged particle beam generation apparatuses to corresponding ones of the different beam irradiation apparatuses.
